# EUROPEAN PATENT APPLICATION

(11) **EP 0 661 027 A1**
(43) Date of publication of application: **05.07.1995**
(21) Application number: 94120335.8
(22) Date of filing: 21.12.1994
(51) Int. Cl.: A61F 5/449

(54) **Ostomy belt**

(30) Priority: 23.12.1993 US 173356
(71) Applicant: Klempner, John, Windsor, ON N9E 1W3 (CA)
(72) Inventor: Klempner, John, Windsor, ON N9E 1W3 (CA)
(74) Representative: Wagner, Karl H., Dipl.-Ing.

(57) **Abstract**

A holder (10) for supporting the weight and restricting the movement of an elongated ostomy bag attachable to a stoma (22) includes a retainer for restraining the bag, preferably a hollow carrier (12) for fittingly receiving the bag, and a fastener (14) for releasably holding the retainer (12) against the abdomen of an ostomate in such an orientation that the bag extends transverse to the body of the ostomate. Supporting the bag transverse to the body prevents dangling of the bag, kinking or compression of the bag when the wearer bends off at the hips, and allows the bag to be worn in conjunction with undergarments and garments having tightly fitting waste bands, and during human sexual activities. The holder (10) increases the ostomate's mobility, improves his or her self confidence and substantially prevents accidental breakage or spillage and, thus, improves the quality of life of the ostomate.

## Description

### Field of the Invention

The invention relates to devices for the collection of matter discharged from an artificial body opening and more particularly to devices for holding an ostomy bag horizontally and close to the body.

### Background Art

Damage to natural body orifices through accident or disease in severe cases requires the creation of artificial openings. The present invention is directed to devices used in connection with an artificial anus or stoma created by way of an ostomy, such as a colostomy or ileostomy, wherein an opening is created in the abdominal wall and connected with the colon or ileum. Unlike the anus, a stoma does not include a sphincter muscle preventing involuntary discharge of bowel contents. Consequently, in most cases, bowel fluid is continuously discharged from the stoma and must be collected in a faecal bag.

Generally, faecal bags are affixed to a base plate which is fastened to the body of an ostomate by mechanical means or adhesive and surrounds the stoma. The base plate and/or the bag can be supported on the body by appliance belt devices such as disclosed in U.S. Patents 2,156,425 by Bowman, 3,532,092 by Rogers, 4,319,571 by Winchell, 4,109,657 by Cartington and Canadian Patent 1,019,216 by Hemrick. Undergarments for the supporting and concealment of ostomy appliances and faecal bags are described in U.S. Patents 3,421,505 by Freeman et al, 4,533,355 by Fair, 4,778,362 by Pollock et al, 4,888,006 by Beaupied, and in Canadian Patent Application 2,023,117 by Larrivée.

Although these supporting devices are generally satisfactory, they all have the major disadvantage that the usually elongated faecal bag is supported on the body in a vertical or downward orientation which promotes the accumulation of stool at the bottom end of the bag and leads to possible spillage or breakage and to a pendulum movement or dangling of the faecal bag, especially when partly or completely filled which severely limits the mobility of the wearer. Even with tightly fitting garments, this effect cannot be prevented. The dangling of the bag is very annoying to the wearer, stresses the connections between the bag, the base plate and the wearer's skin and represents a serious impediment to the enjoyment of sports by ostomates. Furthermore, leakage or breakage of the bag is frequently observed with these prior art supporting devices when the wearer is sitting down, since the bag is then folded and compressed in the groin area. Also, ostomates having their stoma located around or close to the waste line have great difficulty wearing garments with elastic or tightly fitting waste bands and/or belts, since these will impede the flow of stool from the stoma into the bag leading to accumulations of bowel contents at the stoma and subsequent skin irritation. Finally, ostomates engaging in sexual activities will be severely handicapped by most of the prior art bag holders. Thus, the lack of appropriately styled and constructed ostomy bag supporting devices has restricted the real and perceived ability of an individual having experienced an ostomy from again enjoying a normal, active life. Ostomates are typically self-conscious about the fitted appliance, feel handicapped by the stoma, have emotional problems caused by the disability to lead an active, normal life. Furthermore, the inability to wear comfortable clothing for an active life-style seriously affects the quality of life of the ostomate.

### Summary of the Invention

It is now an object of this invention to provide a device for the holding of an ostomy bag which can be worn at all times, improves the quality of life of a ostomate and improves the ostomate's self image and confidence.

It is another object to provide a device which supports the weight of the bag and holds it close to the body of the ostomate in a manner which prevents a pendulum movement of the bag, does not cause folding of the bag when the wearer bends off at the hips, and can be worn in conjunction with undergarments and garments having tightly fitting waste bands or without any clothing at all.

It is yet a further object to provide an ostomy bag supporting device which holds the bag in an orientation transverse to the body of the ostomate.

It is still another object to provide an ostomy bag holder which does not interfere with human sexual activities.

These objects are achieved with an ostomy bag holder which supports the bag against the body of a ostomate in an orientation transverse to the body and preferably spaced from the belt line of the ostomate. A dangling of the bag is thereby prevented. Furthermore, when the bag no longer crosses the belt line in its supported condition, leakage due to compression or folding of the bag is also substantially prevented. By holding the bag tightly against and transverse to the body neither the holder nor the bag interfere with the mobility or sexual activities of the ostomate.

Accordingly, the invention provides a holder for supporting the weight and restricting the movement of an ostomy bag attachable to a stoma, comprising:
a retainer for restraining the ostomy bag when attached to the stoma; and
fastening means for releasably holding the retainer against the body of an ostomate such that the ostomy bag is supported in an orientation transverse to the ostomate's body.

The retainer can be directly affixed to the ostomy bag, for example in the form of retaining bands adhesively applied to the bag and connectable with the fastening means, can be integral with the ostomy bag, or can be constructed to receive the bag.

In a preferred embodiment, the retainer is a hollow carrier for receiving and preferably completely enclosing the ostomy bag.

The carrier preferably has a body engaging surface for placing against an ostomate's abdomen and an aperture in the body engaging surface for receiving a discharge receiving end of an inserted ostomy bag. In a preferred embodiment, the carrier further includes a reversibly closable opening for the insertion of an ostomy bag into and removal from the carrier so that the carrier can be reused. It is preferred that the aperture is connectable with the opening of the carrier in order to permit insertion into and removal from the carrier of the ostomy bag while attached to the stoma of the wearer. This facilitates the use of the holder since then the bag does not have to be threaded through the aperture. The carrier is preferably made of elastically stretchable material to fittingly receive ostomy bags of different size and different degrees of filling. Although the carrier can be made of a net-like material for maximum flexibility, it is preferred that a carrier material be used which completely conceals the ostomy bag so that the ostomate is not embarrassed when wearing the ostomy bag holder in public without any covering clothing. It is also preferred that the carrier material be a soft, flexible fabric to reduce skin irritation. The preferred material is a stretchable cotton fabric which is easily washable and comfortable when worn on the skin and tightly encloses and holds a bag inserted into the carrier. The bag insertion and removal opening of the carrier is preferably provided with cooperating portions of mutually adhearable fabric such as loop and hook-type fasteners, a zipper, or snap fasteners for repeated opening and closing of the carrier. In the preferred embodiment, the fastening means is a belt which is fastenable to opposite ends of the carrier. For improved wear and comfort, the belt is preferably elastically stretchable and/or adjustable in length.

### Brief Description of the Drawings

Figures 1A and 1B show an ostomy bag holder in accordance with the invention respectively worn above and below the belt line depending on the location of the stoma;
Figure 2 is an elevational view of the outer side of the embodiment shown in Figs. 1A and 1B;
Figure 3 is an elevational view of the inner, body engaging side of the embodiment shown in Figs. 1A and 1B; and
Figure 4 illustrates the preferred ostomy bag holder in an opened condition.

### Detailed Description of the Preferred Embodiments

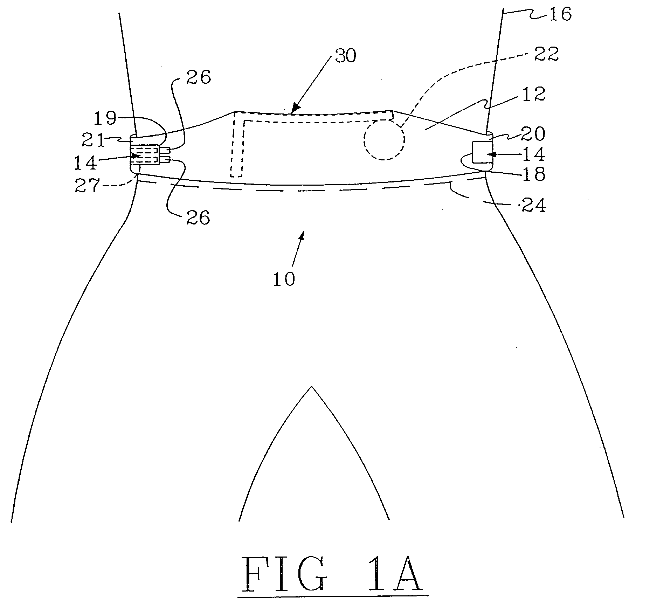

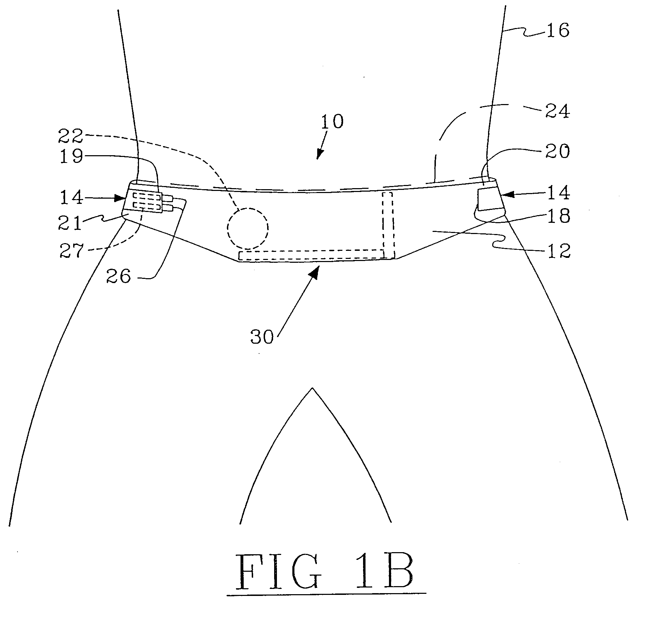

A preferred ostomy bag holder in accordance with the invention as shown in Figures 1A and 1B is in the following generally referred to by reference numeral 10. Holder 10 includes an elongated and pouch-like hollow carrier 12 for an ostomy bag (not shown), which carrier has opposite ends 20, 21, and a fastening belt 14 which has first and second ends 18, 19 and holds the carrier against the body 16 of a person having a stoma (22), i.e. an ostomate.

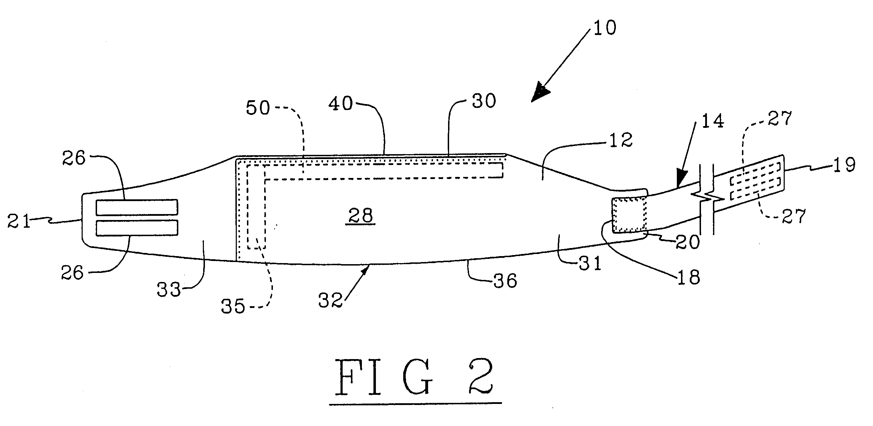

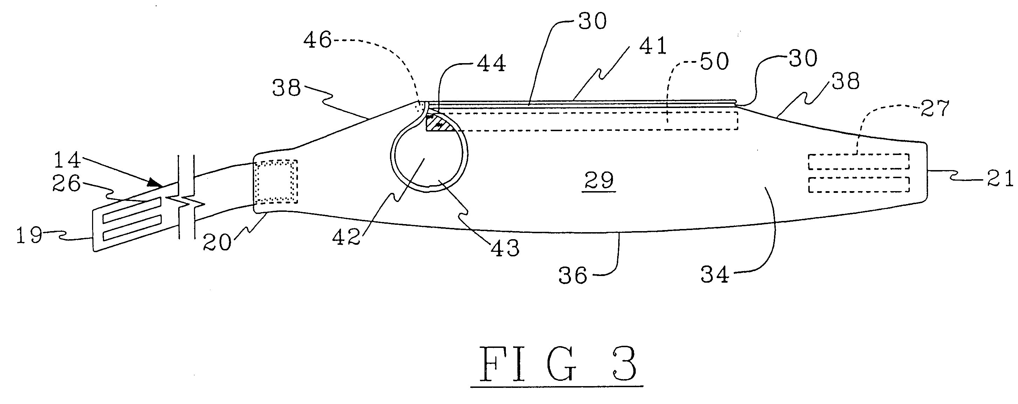

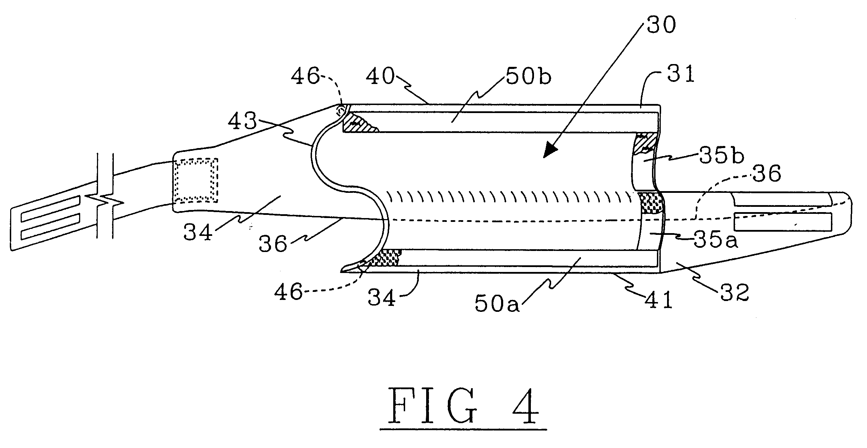

The first end 18 of the fastening belt 14 is permanently affixed to one end 20 of the elongated carrier 12 and is sufficiently long so that the second end 19 overlaps the opposite end 21 of the carrier when the belt is placed around the body 16. The second end 19 is removably connectable to the opposite end 21 of the carrier by two pairs of mutually cooperating fastener strips 26, 27 which allow adjustment of the holder to fit the bodies of different ostomates. The connection between the belt 14 and the carrier 12 and the type of cooperating strips used are discussed in more detail below. The carrier 12 has an opening 30 which can be repeatedly opened and closed for the insertion or removal of an ostomy bag (not shown) and which is shown in the closed condition in Figures 1A and 1B. The holder can be worn with the opening directed upward as shown in Figure 1A or downward as illustrated in Figure 1B. The orientation of the carrier 12 and the opening 30 depends on the preference of the ostomate wearing the holder 10 and on the location of his or her stoma in relation to the belt line 24. However, the holder 10 is preferably worn spaced apart from the belt line 24. The opening 30 will be further discussed below with reference to Figure 4. The carrier 12 and the fastening belt 14 each have an outer side 28 which is illustrated in Figure 2 and an inner, body engaging side 29 which is illustrated in Figure 3.

Turning now to Figure 2, the affixed end of the fastening belt 14 is permanently attached by stitching to the outer side 28 of the carrier 12 at the first end 20. The cooperating strips 26, 27 are bands of mutually adhearable fabric which are respectively sewn onto the inner side 29 of the fastening belt 14 at the second end 19 and the outer side 29 of the carrier 12 at the opposite end 21. In this embodiment, the cooperating strips 26, 27 are made of hook and loop type fasteners, which are commercially available under the trade mark VELCRO. As will be apparent, any permanent and/or adjustable overlap of the fastening belt 14 and the carrier 12 is positioned on the outer side 28 of the holder 10 to minimize irritation of the wearer's skin. Furthermore, the fastening belt 14 is made of a material which is elastic in longitudinal direction so that breathing movements and other expansion and retraction movements of the wearer's abdomen can be compensated by the fastening belt when tightened around the wearer's body 16. The carrier 12 is made of substantially trapezoid front and back panels 32 and 34, the back panel 34 being shown in Figure 3, which panels are made of an elastic cotton fabric. The panels are sewn together along their base edges 36, at the ends 20 and 21, and along their inclined upper edges 38. The respective top edges 40, 41 of the panels 32, 34 are not permanently connected to leave an opening 30 for insertion and removal of an ostomy bag which opening is closable by mutually cooperating fastener strips 50. To increase the size of the opening 30, the front panel 32 is cut from the top edge 40 and into a larger section 31 and a smaller section 33. These sections overlap at their mutually adjacent ends, and are releasably attached to each other by a hook and loop-type fastener 35 commercially available under the trade mark VELCRO.

Figure 3 illustrates the construction of the preferred embodiment on the body engaging side 29. The one piece rear panel 34 is provided with a permanent aperture 42 which is located in close proximity to the top edge 41 of the rear panel. A narrow band of fabric 44 extends between the edge 43 of the aperture 42 and a top edge 41 of the rear panel 34. The band 44 is cut for connection of the aperture 42 with the top edge 41 and, thus, the opening 30, which permits the insertion into and removal from the carrier 12 of an ostomy bag attached to a stoma. A snap fastener 46 is provided for releasably connecting the ends of band 44.

In Figure 4, the carrier 12 is shown in an opened condition wherein the snap fastener 46 is opened, the top edges 40, 41 of the front and back panels 32, 34 and the overlapping ends of the front panel sections 31, 33 are detached from each other, and part of the rear panel 34 is folded over the base edge 36. The mutually adhearable portions 35a, 35b and 50a and 50b of the fastener strips 35 and 50 which releasably connect the top edges 40, 41 and the overlapping portions of the front panel sections 31, 33 respectively, are exposed in this condition.

In use, an ostomy bag attached to the stoma of an ostomate is inserted into the holder 10 in the opened condition illustrated in Figure 4, whereby the orientation of the carrier 12 is selected so that the opening 30 is either upwardly or downwardly directed once the holder is fastened to the ostomate's body and that the opened edge 43 of the aperture 44 is located close to the discharge receiving end of the bag. Then, the ends of edge 43 of the aperture 44 are connected by the snap fastener 46 and the opening 30 is closed by fastener strips 35 and 50. Finally, the carrier is fastened to the abdomen of the ostomate's body 16 by tightening the fastening belt 14 around the abdomen and attaching the free end 19 of the belt to the second end 21 of the carrier 12 by way of fastener strips 26, 27. After that, the ostomate can actively participate in sports and leisure activities and even enjoy sex without the embarrassment of a dangling or jumping ostomy bag filled with stool and visible to others.

Although the invention was described in detail with reference to a particular preferred embodiment, other embodiments in accordance with the invention are conceivable. Instead of a pouch-like structure, the retainer can be in the form of a netlike carrier, or supporting and reinforcing bands adhered or welded to the ostomy bag to which the fastening belt is attachable. These supporting and reinforcing bands can also be integrated into the bag's construction. Both ends of the fastening belt can be releasably attached to the retainer through hooks and eyelets respectively affixed to the belt and the retainer, hook and loop type fasteners, snap fasteners, buttons and a row of button holes, etc. The releasably closable opening 30 of the carrier 12 can be provided not only along the top edges 40, 41 but also along the base edges 36 or along both. The permanent aperture 42 can be positioned at either end of the top edge 41 or base edge 36. In the alternative, a pair of openings 30 and a pair of apertures 42 can be provided to make the holder universally usable by all ostomates irrespective of the stoma's location. For maximum comfort, it is preferred that the whole holder is longitudinally elastically stretchable. However a fastening belt of fixed length can also be combined with at least one longitudinally stretchable band to achieve the same effect. The opening 30 can be maintained in a closed condition by hook and loop type fasteners, buttons, snap buttons or a zipper. The carrier 12 can be made of a single piece of fabric, however using front and back panels facilitates manufacture of the holder. The panels 32, 34 can have a shape other than trapezoid. Ostomates who use bags that can be drained while attached to the stoma will likely prefer wearing a holder 10 having the opening 30 directed downward.

Changes and modifications in the specifically described embodiments can be carried out without departing from the scope of the invention which is intended to be limited only by the scope of the appended claims.

## Claims

1. A holder for supporting the weight and restricting the movement of an elongated ostomy bag insertable into the holder and having an attachment portion attached to a stoma and a discharge receiving portion collecting fecal matter discharged from the stoma, the holder comprising:
an elongated, hollow carrier for supporting the discharge receiving portion of the ostomy bag, the hollow carrier including an elongated body engaging surface for placement against the ostomate's abdomen, the body engaging surface having upper and lower longitudinal edges and an aperture for placement over the stoma, the aperture being provided in the body engaging surface adjacent the upper longitudinal edge and spaced apart from a transverse center line of the elongated hollow carrier; and
means for releasably holding the elongate, hollow carrier against the abdomen of an ostomate in an orientation transverse to the ostomate's body such that fecal matter collected in the discharge receiving portion of the ostomy bag is located to a side of and below the stoma while the body of the ostomate is in a normal, upright position.

2. The ostomy bag holder as defined in claim 1, wherein the carrier further comprises a reversibly closeable opening for insertion of an ostomy bag into the carrier and removal therefrom, to permit reusing of the holder.

3. An ostomy bag holder as defined in claim 1, wherein the aperture is connectable with the closable opening to permit insertion and removal of the discharge receiving portion from the carrier while the ostomy bag is attached to the stoma.

4. An ostomy bag holder as defined in claim 1, wherein the carrier is made of elastic material for fittingly receiving ostomy bags of different sizes and different degrees of filling.

5. An ostomy bag holder as defined in claim 1, wherein the carrier completely encloses any unattached portions of the ostomy bag and is made of a nontransparent material for completely concealing the bag.

6. An ostomy bag holder as defined in claim 2 or 3, wherein the carrier includes cooperating portions of mutually adhearable fabric for reversibly closing the opening, the mutually adhearable fabric portions being respectively affixed to opposite edges of the opening.

7. An ostomy bag holder as defined in claim 2 or 3, wherein the carrier includes cooperating portions of a hook and loop fastener for reversibly closing the opening, the mutually adherable portions being respectively affixed to opposite edges of the opening.

8. An ostomy bag holder as defined in claim 1, wherein the retainer has first and second ends and the fastening means is a belt having a pair of opposite ends respectively attachable to the first and second ends of the retainer.

9. An ostomy bag holder as defined in claim 1, wherein the carrier has first and second ends and the fastening means is a belt having a pair of opposite ends respectively attachable to the first and second ends of the carrier.

10. An ostomy bag holder as defined in claim 9 wherein the belt is made of a longitudinally elastic material for tightly holding the carrier to abdomens of different circumference and for accommodating breathing movements and other expansion and retraction movements of an abdomen engaged by the holder.
